# EUROPEAN PATENT SPECIFICATION

(11) **EP 0 551 925 B1**
(45) Date of publication and mention of the grant of the patent: **26.03.1997**
(21) Application number: 93101183.7
(22) Date of filing: 14.04.1987
(51) Int. Cl.: A41F 9/02, A61F 13/56

(54) **Article including segment which is elastically shirrable after manufacture**
Artikel mit einem Abschnitt, der nach der Herstellung elastisch schrumpfbar ist
Article comportant un segment élastiquement contractable après fabrication

(30) Priority: 15.04.1986 US 852053
(43) Date of publication of application: 21.07.1993
(62) Divisional of application: 87303240.3
(73) Proprietor: THE PROCTER & GAMBLE COMPANY, Cincinnati, Ohio 45202 (US)
(72) Inventor: Baird, James Clark, Cincinnati, Ohio 45223 (US); Koger, II., Thurman J., Hamilton, Ohio 45015 (US); Muckenfuhs, Delmar Ray, Middletown, Ohio 45044 (US); Spahni, Milton Daniel, Okeano, Ohio 45053 (US)
(74) Representative: Bottema, Johan Jan

(56) References cited:
- DE-A- 2 643 858
- US-A- 4 063 559
- US-A- 4 552 795

## Description

### TECHNICAL FIELD

The present invention relates to an article which includes at least one segment which can be elastically shirred subsequent to its manufacture.

The present invention has still further relation to an elastically shirrable segment per se. The segment preferably includes a prestretched and tensioned elastomeric member which is secured in fixed relation to at least one rigidifying member. The composite structure thus formed is strong enough to resist collapse in a direction parallel to the tensile forces acting upon the prestretched and tensioned elastomeric member at least until the attachment of the segment to the article has been completed.

### BACKGROUND OF THE INVENTION

The fit of a garment to the body of the wearer is one of the key aspects of clothing design. Garment fit is critical for several reasons. First, garments that fit well are aesthetically pleasing to the wearer, as well as to others. Second, clothing that fits the body well does not hinder body movement. For instance, clothing that is too tight will prevent the body from undergoing its normal muscular expansions and contractions, causing discomfort to the wearer. Clothing that is too loose can hinder body motion by entangling the body in the garment or by adding unwanted bulk. Third, good fit often provides the function of garment securement. For instance, waist bands hold pants up, hat bands hold hats on, and some cuffs hold sleeves or pant legs in place. Fourth, there is a kind of fit that seals the environment beneath the clothing from leaking to the outer environment, or vice versa. This function is obvious in durable garments such as rainwear or cold weather clothing, and in disposable garments such as disposable diapers.

Elastics of many forms are often used to provide one or more types or garment fit. The forms of these elastics include composite materials such as those used in undergarment waist bands, and homogeneous elastomeric materials such as the waist and legbands found in many disposable diapers. There are also linear, and two-dimensional stretch elastics used in clothing. Waist bands, and elastic cuffs are considered linear, whereas in pantyhose the material stretches in two dimensions to provide a contoured body fit.

A common problem with elastics on factory manufactured articles, such as clothing, is that the amount of tension the elastic applies against the body is not right for each individual wearer. This problem arises because factory made clothing is manufactured in certain discrete sizes. While the elastic tension may be right for a person having dimensions in the middle of a particular size range, the tension may be too light for a slightly smaller person or too great for a somewhat larger person. If the tension is too light the garment may droop, while if too great, the elastics may leave red marks on the skin and cause discomfort.

Achieving garment fit using elastics also poses problems for the manufacturer of garments. First, attaching elastic materials to a garment, especially when the elastic is in a prestretched condition, requires somewhat complex material handling methods. Fixturing is often required to hold the elastic in a stretched condition, or the garment in a shirred or gathered condition while the attachment is made. This extra handling and fixturing can slow down automated production lines. Secondly, once the elastic is attached to the garment and tension is released, the garment shirrs in the area of the elastic making the garment unwieldy as it is passed either on to the next step in the manufacturing process or to a packing operation.

Prior to the development of the materials of the present invention, the problems associated with garment elastics have generally been dealt with in two basic ways. In particular, the problem of achieving the right amount of tension for the individual wearer has typically been accomplished by providing multiple fastening locations. These allow the elastic to be stretched different amounts as the garment is fastened to the body. A simple example of this is an elasticized belt for trousers that includes multiple fastening points at the belt buckle. This allows the wearer to select a wide range of waist band tensions. Another example of this is in disposable diapers having an elasticized waist band and tape fasteners. In this instance, the amount of tension in the waist band elastic can be controlled to some degree by the tension the mother applies to the waist band elastic before the tape fasteners are secured. While some degree of tension adjustment is afforded by this method, it is difficult for the person applying the diaper to precisely adjust this tension while the baby is squirming. This method of diaper elastic tensioning also compromises the position of tape attachment from the ideal. For instance, if the elastic is stretched to a great extent to achieve the desired tension, the tape fastening points may be far enough from their ideal location that the overall diaper fit becomes distorted. This distortion may cause poor fit in other critical areas, for example the leg band area.

The problem of assembling garment shirring elastic components which are not in tension when applied has typically been addressed in high speed manufacturing lines by the use of heat shrinkable elastics. These elastics are designed to be attached to a garment such as a disposable diaper while they are in the relaxed state or under low tension. After they are attached, heat is applied to the elastic at some point during or subsequent to the manufacturing process. Upon heating, these elastics contract and regain much of their original elasticity.

These heat shrinkable elastics are manufactured in several forms. Some are homogeneous materials. These are typically thermoplastic elastomers that were stretched to orient their molecular structures after casting. When they are heated after assembly in the diaper, they shrink back, losing some of their orientation. Other heat shrinkable elastics are composite structures such as those disclosed in U.S. patent 4,552,795 issued to Hansen et al. on November 12, 1985. The structures disclosed by Hansen et al. are preferably comprised of prestretched elastomeric strands that are laminated between two relatively inelastic strips of film with inelastic thermoplastic polymer. Upon the application of heat the thermoplastic polymer softens, allowing the elastic member to move relative to the outer layers, thereby causing the outer layers and the article to which they are secured to elastically contract and shirr. Thus, if this laminate is attached to a portion of a garment, say a diaper waist band, the result upon heating is garment shirring in proportion to the relative movement between the elastic member and the outer layers of the laminate.

While solving many of the problems of elastic material factory assembly, the application of heat required to activate such prestretched and tensioned elastics may, in some circumstances, adversely affect other components in the article to be elasticized. Furthermore, such heat activatable materials do not help in a reasonable way in those situations where elastic adjustment by the consumer is desired. Heat activation by the consumer is impractical because it requires a heat source that is usually unavailable to the consumer, it is potentially dangerous, and it is difficult to reproducibly control without standardized processing conditions and equipment.

Accordingly, it is an object of the present invention to provide elastic materials which avoid the foregoing problems altogether.

It is another object of the present invention to provide an article which can be applied to the wearer while it is not in tension and thereafter elasticized.

### DISCLOSURE OF THE INVENTION

The elastic materials of the present invention are composite structures. A simple, exemplary embodiment of this composite structure can comprise a three layer laminate. To further describe this structure it is easiest to describe it in terms of a preferred method of manufacture for a specific embodiment.

The first step is to select as a starting material an elastomeric band. While there are many different material and size combinations possible for this band, for purposes of illustration let it be assumed that the band is 12.7 mm wide, 254 mm long, and 0.127 mm thick. This band can be comprised of nearly any elastomeric material, synthetic natural rubber being particularly well suited in situations where long periods of time are likely to pass before the tension in the elastomer is to be released.

The next step is to stretch the band in at least one direction. For example, it can be stretched to 3 times its original length. The band is then preferably clamped at each end to hold it in its outstretched condition. Next, the other two rigidifying layers of the laminate are applied to the stretched band. These other two rigidifying layers may be of identical composition and are preferably comprised of a relatively rigid, brittle material, such as extrusion cast polystyrene. The polystyrene rigidifying layers can be relatively thin, i.e., a thickness of 0.025 mm is sufficient for the exemplary band stretched to 3 times it original length. The rigidifying layers preferably have the same planar dimensions as the outstretched rubber. These polystyrene layers are placed on the top and bottom of the stretched rubber forming a sandwich. These three layers are then heat sealed together under pressure, thereby forming a thermally bonded laminate. After the laminate has cooled, the clamps are removed from the ends of the rubber. Upon clamp removal, the planar laminate structure (exclusive of those portions held in the clamps) remains substantially the same length as the stretched elastic rubber was while it was constrained by the clamps prior to lamination. The resultant laminate is relatively flexible and can easily be handled without maintaining it in tension. The entire laminate band or a segment cut therefrom can be secured to any desired article to be elasticized. For example, the ends of a segment having a length of about 127 mm could be attached to the opposed portions of an adjustable hat by sewing, riveting etc.

It is, of course, recognized that it is not necessary in the practice of the present invention for the entire elastically shirrable segment to comprise a laminate composite structure of the type described earlier herein. For example, the elastically shirrable segment may include one or more such isolated laminate composite structures along its length. Release of tension in any one of the composite structure portions of the segment will shirr that portion of the article to which the ends of the segment are secured, i.e., release of tension in any portion of the segment will draw the ends of the segment closer to one another

### BRIEF DESCRIPTION OF THE DRAWINGS

While the specification concludes with claims particularly pointing out and distinctly claiming the present invention, it is believed the present invention will be better understood from the following description in conjunction with the accompanying drawings in which:

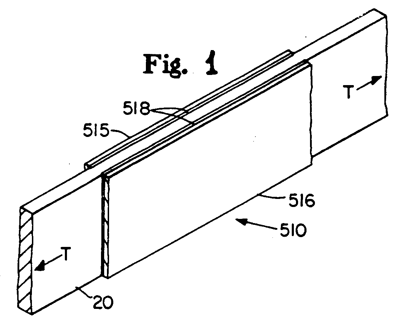

Figure 1 is a simplified perspective illustration of an alternative elastically shirrable segment of the present invention which is self-activating so as to automatically shirr the article to which it is secured before the article is placed in service.

### DETAILED DESCRIPTION OF THE INVENTION

Elastically shirrable structures of the present invention may be formed in many different configurations using a variety of materials. Exemplary embodiments utilizing differing materials of construction and differing configurations will be disclosed herein for purposes of illustration only. Various changes and modifications to the exemplary embodiments can be made without departing from the scope of the invention. Accordingly, these exemplary embodiments are not intended to limit the present invention, as described in the appended claims.

### Materials of Construction

Composite elastically shirrable structures of the present invention are typically comprised of up to three material types. These are the elastomeric material, the rigidifying material, and an optional intermediate material such as an adhesive which may be used to attach the elastomeric material to the rigidifying materials(s). These three types of materials are discussed separately hereinafter.

### The Elastomeric Material

Preferably, the elastomeric material is a material that can undergo high levels of reversible strain. Elastomers that can be stretched to two or more times their original length and then recover to their original length once the stretching force is removed are particularly useful for the purpose of creating garment shirring. However, elastomers that cannot be reversibly stretched as far may find utility in some applications. Even elastomers which exhibit a degree of irreversible stretch may be utilized, depending upon the particular application.

Elastomers that will maintain a fixed tension when they are stretched and held for long periods of time (perhaps a year) are particularly preferred in situations where long periods of time may pass between the manufacture and use of the elastically shirrable article. Generally these preferred materials are comprised of thermoset rubbers, such as synthetic natural rubbers. Elastomers that will not maintain tension for a long period of time will have more limited utility in practicing the present invention. Their principal use would be in applications where the elastic is to be activated soon after lamination of the composite structure is complete (such as activation on line in a manufacturing plant) or in applications where variable and limited elastic recovery is acceptable. Elastomers that will not maintain tension for extended periods of time are generally comprised of thermoplastics, such as ethylene vinyl acetate copolymer.

### The Rigidifying Material

The term "rigid", for the purpose of this invention, is a relative term. It means that the rigidifying material will not foreshorten enough to allow the compressive forces exerted by the stretched elastomer to return the stretched elastomer to its original untensioned length. That is, it is relatively inelastic when compared to the elasticity of the elastomeric component in a given laminate composite structure. Materials such as polystyrene, blends of polystyrene and polyethylene, polyethylene laminated to paper, and surlyn have all been used as a rigidifying component in exemplary elastically shirrable segments of the present invention. These materials all have very different moduli of elasticity from one another, but used appropriately (the right thicknesses, relative material widths, elastic pretension, etc.) they all can work acceptably as a rigidifying member in elastically shirrable segments of the present invention.

The rigidifying member may also be brittle or not. The decision to choose a brittle material over a ductile material depends upon the method of elastic activation desired in the elastically shirrable segment. If it is desirable to activate the elastic by wiggling the composite to cause cracks and delamination in the rigid layer or layers, then a material brittle at the temperature of use is preferred. If however, the elastic is activated by stripping or peeling off the rigid layer from the composite structure, then a more ductile rigidifying material is preferred.

In a yet another embodiment of the present invention, the rigidifying member or layer could be a durable material like steel. For example, it could be a permanent component of a machine that applies the prestretched and tensioned elastic to the garment. In this case, the elastic would be stretched and adhered to a permanent rigid layer such as an endless, flexible steel conveyor band. The resultant laminate comprising the steel conveyor band having the prestretched and tensioned elastomeric member adhered thereto would then be brought into contact with the garment or other article to be elasticized and the elastomeric member would be affixed to the article. Finally, the permanent rigidifying layer would be stripped away leaving the prestretched and tensioned elastomeric member adhered to the article as the article moved downstream. Such a method may be particularly useful for attaching stretched elastic leg bands to a continuously moving web of disposable diapers.

Rigidifying members of the present invention may have many different material configurations. For instance, it could be a flat film, an embossed flat film, a nonwoven fabric, a hollow tube, a rigid foam, a scrim, a laminate of several materials or a molded shape. The materials could have a wide range of thickness, depending upon the tension in the prestretched elastomeric member, and could even be variable in thickness throughout the width and/or length of the composite structure. The rigidifying member or members could also be an integral component of the article to be elasticized rather than an independent element.

### The Optional Intermediate Material

The use of an intermediate material to secure the elastomeric member and the rigidifying member to one another is optional in constructing elastically shirrable segments of the present invention. As will also be pointed out in subsequent sections of this specification, it is not always necessary for the rigidifying members to be secured along their length directly to the prestretched elastomeric member. However, in those situations where an intermediate material is employed, it most typically comprises an adhesive. In this capacity, it serves to bond the prestretched and tensioned elastomer to the rigidifying member. This is especially valuable where a natural heat seal bond between the prestretched elastomer and the rigidifying layer is either too strong or too weak. In this case, the adhesive must be selected so as to give the right adhesive forces and so as not to detract from the function of the composite structure.

The optional intermediate material may also comprise more than just an adhesive. It may have considerable bulk relative to the prestretched elastomer and/or rigidifying layer(s). One such example of a composite structure of the present invention could comprise a multiplicity of prestretched elastomeric strands running parallel to a multiplicity of rigidifying strands, both materials enveloped by a matrix comprised of a third material, such as a foam. In this embodiment, the foam must exhibit sufficient adhesive and mechanical strength to hold the composite structure together under the tension of the prestretched elastomeric strands, but be weak enough to collapse with the elastomer when the rigidifying strands are broken. This type of structure may have particular utility as a replacement for durable garment elastics.

In many embodiments of the present invention, an intermediate material is not necessary. However, when the optional intermediate material is not present, it is still a requirement that the prestretched elastomeric member and the rigidifying member be secured in fixed relation to one another so as to form a composite structure which is strong enough to resist collapse in a direction parallel to the tensile forces acting upon the prestretched elastomeric member prior at least until the attachment of the segment to the article has been completed. Methods for securing the prestretched elastomeric member and the rigidifying member directly to one another without use of an intermediate material include heat sealing, solvent bonding (e.g., as by placing a solvent for one or both materials between the layers, and then driving off the solvent), solution casting one layer onto the other, and mechanical interlocking. Bonds made without the optional intermediate layer must also be strong enough to hold the prestretched elastomer in its full, outstretched condition before activation, and weak enough to fail upon whatever form of activation is desired, preferably mechanical manipulation of the composite structure.

### Possible Uses for the Elastically Shirrable Segments

Elastically shirrable segments of the present invention can be applied to many garments and other articles where gathering or shirring is needed. They can be applied for the purpose of article shirring to both disposable garments and durable garments. In addition, they can be applied to disposable and durable articles where elastic tensioning, is desired. The following list sets forth illustrative examples of such potential applications:

| 1. Disposable Garment Examples | |
|---|---|
| | Advantage |
| b. Disposable Sanitary Hair Nets or Surgical Caps | To provide the ideal tension for hat securement without causing red marking on the skin |
| c. Disposable Sanitary Gloves | To provide a means of securement for non-elasticized plastic gloves |

| 2. Durable Garment Examples | |
|---|---|
| b. As a manufacturer's replacement for garment elastics which must be applied in a stretched condition | Allows the manufacturer to sew or otherwise attach the elastically shirrable segments while they are in a substantially untensioned condition. |
| c. As a consumer applied replacement for garment elastics which must be attached in a stretched condition | Allows the home tailor to apply the elastically shirrable segments while they are in a substantially untensioned condition. |

| 3. Non Garment Examples | |
|---|---|
| b. Automobile Seat Covers | Allows easier installation of seat covers by eliminating the need to fight the elastics while hooking the covers in place. |
| c. Disposable bed sheets | Allows easier installation of corners which are elasticized only after they are properly in place. |

Beyond unidirectional article shirring applications, such as the exemplary applications set forth above, there are still other uses for elastically shirrable segments of the present invention. For instance, elastically shirrable segments of the present invention can also be employed to release elastic forces in more than a single direction, i.e., in two or more directions. Embodiments of the latter type use starting materials similar to those described earlier herein, except that before applying the rigidifying member, the elastomeric member is prestretched in tension in two or more directions instead of one. The result is an elastically shirrable segment that when applied to a garment or other article will draw in or shirr an initially planar portion of the article in two or more directions rather than in just one direction, as in the unidirectional examples described earlier herein.

### Preferred Ways of Incorporating Elastically Shirrable Segments into An Article

While not intended to be an exhaustive listing, the following are illustrative of ways to attach elastically shirrable segments of the present invention to a garment or other article:
1. Heat Bonding
   a. With or without an adhesive
   b. Discrete bonds, or continuous bonding along the length of the composite structure
2. Ultrasonic Bonding
   a. With or without additional heat
   b. With or without adhesive
   c. In discrete or intermittent patterns
3. Mechanical Attachment
   (e.g. within a tubular portion of the garment with the elastic knotted at the ends, by sewing, with staples, etc.)
4. Using Adhesive
   (e.g., hot melt, cold set, pressure sensitive, and contact adhesives)

In selecting a particular means of attachment to the garment or other article, it is in most cases preferable to attach the garment or article to be elastically shirred securely to the prestretched elastomeric component in the composite structure rather than to the rigidifying layer. This is generally so because in order for the composite to release tension in the prestretched elastomeric member, relative movement must occur between the prestretched elastomeric member and the rigidifying member. In the most extreme case, the rigidifying member is completely stripped away. Therefore, in many applications it is highly desirable to have the prestretched elastomeric member exposed in some portions of the composite for the purpose of providing points or areas for attachment to the garment or other article to be elastically shirred.

### Example

### Solid Seal Configuration Which is Self-Activating Prior to End Use

In certain instances it may be desirable that the release of tension in the prestretched elastomeric member of composite structures of the present invention automatically occur without mechanical manipulation of the composite structure. Materials of the latter type are referred to as self-activating, i.e., the elastic composite could be applied to an article while in a substantially untensioned condition, but arrive in the end user's hands in an elastically shirred condition due to the self-activation which takes place after attachment of the elastically shirrable segment to the article or garment in question.

The composite structure embodiment which is illustrated in simplified perspective in Figure 1 comprises a layer of masking tape 515,516 continuously secured to each side of a stretched rubber 20 while it is subject to tension "T", thereby forming a trilaminate composite structure 510 of the present invention.

### Materials:

Rubber (20) - Fulflex 9411, 12.7 mm wide by 0.178 mm thick, as available from Fulflex Inc., Bristol, R.I. Rigidifying Members (515,516) - Spectape ® pressure sensitive masking tape, as available from Spectape, Inc., Erlanger, Kentucky.

### Procedure:

A 150mm long piece of rubber 20 was extended to 3 times its original untensioned length and a layer of the pressure sensitive masking tape 515, 516 was adhered to each side of the prestretched rubber by means of the pressure sensitive adhesive 518 on the tape, as generally shown in Figure 7. The trilaminate structure 510 thus formed was pressed together by hand. The overlapping edges of the tape were trimmed so as to equal the width of the stretched rubber 20 and cut to the same overall length as the stretched rubber. The resultant laminate composite structure 510 maintained the stretched rubber in its fully extended condition for only a short time when tension "T" on the segment was released. It was observed that the stretched rubber 20 began to slowly contract. After about an hour had passed, the tapes 515, 516 had shirred along substantially all of their length, and the rubber 20 had returned to its original untensioned length.

Laminate composite structures of this type find particular utility where it is desired to apply the composite to an article or garment while in a substantially untensioned condition, yet provide the end user with an elastically shirred article which is ready for immediate use.

## Claims

1. An elastically shirrable segment for attachment to an article to be elastically shirred, a predetermined portion of said shirrable segment comprising an elastomeric member (20) which, prior to and during manufacture of said article, is maintained in a prestretched and tensioned condition in the desired direction of shirring, said elastomeric member (20) continuously secured in its prestretched and tensioned conditions in a substantially fixed relation to at least one rigidifying member (515, 516) by means of adhesive (518) to form a composite structure (510), which is strong enough to resist collapse in a direction parallel to the tensile forces acting upon said prestretched and tensioned elastomeric member at least until the attachment of said segment to said article has been completed, but which begins to slowly contract a short time after the tension on said elastomeric member is released,
characterised in that
said segment exhibiting an ability to automatically elastically shirr along a predetermined portion of its length subsequent to its attachment to said article, said segment being automatically elastically shirred by self-induced relative movement between said prestretched and tensioned elastomeric member (20) and said rigidifying member, (515, 516) whereby a degree of shirring of said segment will occur in the direction of prestretching of said elastomeric member (20) said degree of segment shirring being proportional to the extent to which there is relative movement between said prestretched and tensioned elastomeric member (20) and said rigidifying member (515, 516) in the area comprising said composite structure.

2. A segment according to Claim 1 wherein said rigidifying member (515, 516) comprises a layer of pliable material.

3. A segment according to either one of Claims 1 and 2 wherein said layer of pliable material comprises a polymeric film.

4. A segment according to any one of Claims 1-3 wherein said adhesive (518) is pressure sensitive.

5. A segment according to Claim 4 wherein said rigidifying member (515, 516) comprises a layer of pressure sensitive tape.

6. A segment according to any one of Claims 1-5 wherein at least a portion of said prestretched and tensioned elastomeric member in said elastically shirrable segment is exposed for securement directly to said article.

7. A segment according to any one of Claims 1-6 wherein said composite structure formed by said prestretched and tensioned elastomeric member and said rigidifying member extends along substantially the entire length of said segment.

8. An article to be elasticised, said article including at least one elastically shirrable segment in accordance with any one of the claims 1-7 wherein opposed ends of said elastically shirrable segment in said article are secured to said article.

## Patentansprüche

1. Ein elastisch kräuselbares Segment zum Befestigen an einem elastisch zu kräuselnden Artikel, wobei ein vorherbestimmter Abschnitt des genannten kräuselbaren Segments einen elastomeren Bauteil (20) umfaßt, welcher vor und während der Herstellung des genannten Artikels in einem vorgestreckten und gespannten Zustand in der gewünschten Kräuselungsrichtung gehalten wird, wobei der genannte elastomere Bauteil (20) in seinen vorgestreckten und gespannten Zuständen in einer im wesentlichen feststehenden Beziehung an mindestens einem versteifenden Bauteil (515, 516) mittels Klebstoff (518) kontinuierlich fixiert ist, um eine Verbundstruktur (510) zu bilden, welche stark genug ist, um einem Zusammenbrechen in einer Richtung parallel zu den Zugspannungskräften, welche auf den genannten vorgestreckten und gespannten elastomeren Bauteil einwirken, zu widerstehen, mindestens bis die Befestigung des genannten Segments am genannten Artikel fertiggestellt worden ist, welches aber kurze Zeit, nachdem die Zugspannung am genannten elastomeren Bauteil freigegeben worden ist, beginnt langsam zu kontrahieren,
dadurch gekennzeichnet,
daß das genannte Segment eine Fähigkeit zum automatischen elastischen Kräuseln entlang einem vorherbestimmten Abschnitt seiner Länge nach seiner Befestigung am genannten Artikel aufweist, wobei das genannte Segment durch selbstinduzierte Relativbewegung zwischen dem genannten vorgestreckten und gespannten elastomeren Bauteil (20) und dem genannten versteifenden Bauteil (515, 516) automatisch elastisch gekräuselt wird, wodurch ein Kräuselungsgrad des genannten Segments in der Richtung des Vorstreckens des genannten elastomeren Bauteils (20) auftreten wird, wobei der genannte Grad an Segmentkräuselung proportional zu dem Ausmaß ist, in welchem es eine Relativbewegung zwischen dem genannten vorgestreckten und gespannten elastomeren Bauteil (20) und dem genannten versteifenden Bauteil (515, 516) in der Zone, welche die genannte Verbundstruktur umfaßt, gibt.

2. Ein Segment nach Anspruch 1, bei welchem der genannte versteifende Bauteil (515, 516) eine Schichte aus biegsamem Material umfaßt.

3. Ein Segment nach einem der Ansprüche 1 und 2, bei welchem die genannte Schichte aus biegsamem Material eine Polymerfolie umfaßt.

4. Ein Segment nach einem der Ansprüche 1 bis 3, bei welchem der genannte Klebstoff (518) druckempfindlich ist.

5. Ein Segment nach Anspruch 4, bei welchem der genannte versteifende Bauteil (515, 516) eine Schichte aus Selbstklebeband umfaßt.

6. Ein Segment nach einem der Ansprüche 1 bis 5, bei welchem mindestens ein Abschnitt des genannten vorgestreckten und gespannten elastomeren Bauteils in dem genannten elastisch kräuselbaren Segment zur Fixierung direkt am genannten Artikel exponiert ist.

7. Ein Segment nach einem der Ansprüche 1 bis 6, bei welchem die genannte Verbundstruktur, welche vom genannten vorgestreckten und gespannten elastomeren Bauteil und vom genannten versteifenden Bauteil gebildet ist, sich entlang im wesentlichen der gesamten Länge des genannten Segments erstreckt.

8. Ein zu elastifizierender Artikel, wobei der genannte Artikel mindestens ein elastisch kräuselbares Segment in Übereinstimmung mit einem der Ansprüche 1 bis 7 inkludiert, bei welchem gegenüberliegende Enden des genannten elastisch kräuselbaren Segments im genannten Artikel am genannten Artikel fixiert ist.

## Revendications

1. Segment apte à se contracter élastiquement, destiné à être fixé à un article qui doit être resserré élastiquement, une partie prédéterminée dudit segment apte à se contracter comprenant un élément en élastomère (20) qui, avant et pendant la fabrication dudit article, est maintenu dans un état préétiré et tendu dans la direction souhaitée de contraction, ledit élément en élastomère (20) étant assujetti d'une manière continue, dans ses états préétiré et tendu et dans une position relative sensiblement fixe, à au moins un élément de rigidification (515, 516) au moyen d'un adhésif (518), afin de former une structure composite (510), qui est suffisamment résistante pour s'opposer à un affaissement dans une direction parallèle aux forces de traction agissant sur ledit élément en élastomère préétiré et tendu, au moins jusqu'à ce que la fixation dudit segment audit article ait été réalisée, mais qui commence à se contracter lentement peu de temps après que la tension exercée sur ledit élément en élastomère a été relâchée,
caractérisé en ce que
ledit segment présente une aptitude à se contracter élastiquement de façon automatique le long d'une partie prédéterminée de sa longueur à la suite de sa fixation audit article, ledit segment se contractant élastiquement de façon automatique sous l'effet d'un mouvement relatif auto-induit entre ledit élément en élastomère (20) préétiré et tendu et ledit élément de rigidification (515, 516), une contraction d'un certain degré dudit segment se produisant alors dans la direction de préétirement dudit élément en élastomère (20), ledit degré de contraction du segment étant proportionnel à la distance sur laquelle a lieu le mouvement relatif entre ledit élément en élastomère (20) préétiré et tendu et ledit élément de rigidification (515, 516) dans la zone renfermant ladite structure composite.

2. Segment selon la revendication 1, dans lequel ledit élément de rigidification (515, 516) comprend une couche de matériau pliable.

3. Segment selon l'une ou l'autre des revendications 1 et 2, dans lequel ladite couche de matériau pliable comprend un film de polymère.

4. Segment selon l'une quelconque des revendications 1 à 3, dans lequel ledit adhésif (518) est sensible à la pression.

5. Segment selon la revendication 4, dans lequel ledit élément de rigidification (515, 516) comprend une couche constituée par un ruban sensible à la pression.

6. Segment selon l'une quelconque des revendications 1 à 5, dans lequel au moins une partie dudit élément en élastomère préétiré et tendu, dans ledit segment apte à se contracter élastiquement, est exposée pour être assujettie directement audit article.

7. Segment selon l'une quelconque des revendications 1 à 6, dans lequel ladite structure composite, formée par ledit élément en élastomère préétiré et tendu et ledit élément de rigidification, s'étend sur sensiblement la longueur entière dudit segment.

8. Article devant être rendu élastique, ledit article comportant au moins un segment apte à se contracter élastiquement selon l'une quelconque des revendications 1 à 7, dans lequel les extrémités opposées dudit segment apte à se contracter élastiquement dans ledit article sont assujetties audit article.
